Europäisches Patentamt

European Patent Office

Office européen des brevets

(19)

(11) Numéro de publication: **0 303 540 B1**

# FASCICULE DE BREVET EUROPEEN

(12)

(45) Date de publication de fascicule du brevet: **12.05.93**

(51) Int. Cl.5: **C12N 15/00**, C12P 21/02, A61K 37/02, C07K 13/00

(21) Numéro de dépôt: **88402078.5**

(22) Date de dépôt: **10.08.88**

(54) **Analogue du facteur VIII, procédé de préparation, et composition pharmaceutique le contenant.**

(30) Priorité: **11.08.87 FR 8711415**

(43) Date de publication de la demande:
**15.02.89 Bulletin 89/07**

(45) Mention de la délivrance du brevet:
**12.05.93 Bulletin 93/19**

(84) Etats contractants désignés:
**AT BE CH DE ES FR GB GR IT LI LU NL SE**

(56) Documents cités:
**EP-A- 0 253 455**
**EP-A- 0 265 778**
**WO-A-86/06101**
**WO-A-87/07144**
**WO-A-88/00831**

**BIOCHEMISTRY, vol. 25, no. 26, 30 décembre 1986, pages 8343-8347, American Chemical Society, Washington, DC, US; D.L. EATON et al.: "Construction and characterization of an active factor VIII variant lacking the central one-third of the molecule"**

**BIOCHEMICAL & BIOPHYSICAL RESEARCH COMMUNICATIONS, vol. 145, no. 1, 29 mai 1987, pages 234-240, Academic Press, Inc.,**

**Duluth, MN, US; A. PAVIRANI et al.: "Two independent domains of factor VIII co-expressed using recombinant vaccinia viruses have procoagulant activity"**

(73) Titulaire: **TRANSGENE S.A.**
**11, rue de Molsheim**
**F-67000 STRASBOURG(FR)**

(72) Inventeur: **Meulien, Pierre**
**73, avenue des Vosges**
**F-67000 Strasbourg(FR)**
Inventeur: **Pavirani, Andrea**
**4, rue Stimmer**
**F-67000 Strasbourg(FR)**

(74) Mandataire: **Ahner, Francis et al**
**CABINET REGIMBEAU, 26, avenue Kléber**
**F-75116 Paris (FR)**

Rank Xerox (UK) Business Services
(3.10/3.6/3.3.1)

EP 0 303 540 B1

**Description**

Le clonage du cADN du facteur VIII (FVIII) (1 – 3) a permis de mieux comprendre la structure et les fonctions de cette molécule complexe.

Le premier produit de traduction du mARN est une molécule de 2.351 acides aminés, comprenant un peptide signal de 19 acides aminés. On observe des homologies internes dans les séquences en acides aminés qui permettent de définir des domaines : un triplet de domaines A, un domaine B unique et un doublet de domaines C, disposés dans l'ordre suivant : A1 – A2 – B – A3 – C1 – C2 (2, 4).

Le domaine B unique est codé par un exon de 3.100 pb (exon 14 du gène FVIII) (5) et contient 19 des 25 sites potentiels de glycosylation (Asn) ; cette partie de la molécule est clivée et perdue lors de l'activation par la thrombine (2, 4, 6).

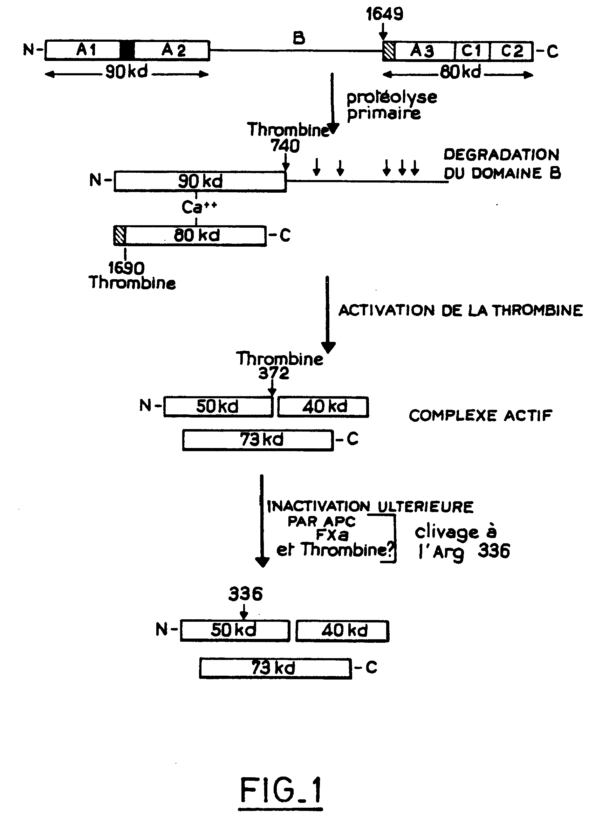

Le modèle actuel du mécanisme de maturation du facteur VIII est schématisé dans la figure 1.

Dans ce modèle, la première activation est due à un clivage de la molécule entre les acides aminés $Arg^{1648}$ et $Glu^{1649}$ ce qui donne un précurseur de la chaîne légère de 80 Kd et un précurseur de la chaîne lourde de > 200 Kd.

Les 2 chaînes sont probablement reliées par un ion métallique ($Ca^{++}$ ?) avant que le clivage ne soit réalisé.

Ensuite le domaine B est dégradé par plusieurs clivages protéolytiques, puis l'activation par la thrombine s'effectue par un clivage entre les acides aminés $Arg^{739}$ et $Ser^{740}$ et entre $Arg^{1689}$ et $Ser^{1690}$ ce qui donne une chaîne lourde de 90 Kd et une chaîne légère de 73 Kd. La nécessité d'un clivage supplémentaire entre $Arg^{371}$ et $Ser^{372}$ pour obtenir une activité maximale est encore controversée ; ce clivage pourrait au contraire déstabiliser la molécule (6, 7).

Il est généralement admis qu'une incubation prolongée en présence de thrombine entraîne une rapide diminution d'activité. D'autres facteurs peuvent également inactiver le facteur VIII comme la protéine C activée et le facteur Xa qui clive la molécule en plusieurs points, en particulier à $Arg^{336}/Met^{337}$ (7).

Dans ce processus de maturation, en remarque que le domaine B ne joue pas de rôle dans l'activité de la molécule mature puisqu'il est perdu après activation par la thrombine. De plus, Toole et al. (8) ont comparé les séquences nucléotidiques des gènes du facteur VIII humain et porcin et ont montré que les 2 domaines B sont très divergents alors que les chaînes lourde et légère sont très conservées.

Ceci suggère (8, 9, 10, 11) qu'une molécule dépourvue de domaine B ne perdrait pas sa fonction procoagulante.

Deux approches ont été suivies pour tester cette hypothèse : soit la délétion, dans le cADN, de grands fragments d'ADN correspondant au domaine B ce qui donne des dérivés du facteur VIII plus courts (8, 9), soit la co – expression dans des cellules de mammifères de fragments d'ADN correspondant aux chaînes lourde et légère (10, 11).

La co – expression dans des cellules de mammifères des chaînes lourde et légère résulte en une production détectable de FVIII:C mais à un niveau 5 fois inférieur à celui obtenu par l'expression de la molécule complète. Ceci suggère que l'association des 2 chaînes est inefficace ce qui diminue l'activité de la molécule (10, 11).

Ces expériences ont montré qu'une molécule dépourvue de la plus grande partie du domaine B mais qui a conservé les sites de maturation correspondant aux acides aminés 740, 1649 et 1690 (qui semblent nécessaires à l'activation) présente une activité procoagulante normale et de plus, a l'avantage d'être exprimée 10 fois plus efficacement dans les cellules de mammifères (8). Cette molécule peut être activée par la thrombine de la même manière que la molécule naturelle entière (9).

Le facteur VIII circulant dans le plasma est associé au facteur von Willebrand (fvW) qui semble le stabiliser ; en effet la demi – vie du FVIII in vivo diminue très fort en absence de fvW (16).

Eaton et al. (9) ont décrit un dérivé du facteur VIII délété dans le domaine B, qui lie efficacement le fvW, ce qui permet d'attendre une demi – vie normale in vivo pour la molécule délétée du domaine B.

La présente invention concerne l'analogue du facteur VIII dont les acides aminés 771 à 1666 ont été délétés. On l'appellera aussi tout au long de la description et des exemples FVIIIΔII.

L'analogue du facteur VIII selon la présente invention est, de préférence, préparé à partir d'une culture de cellules eucaryotes produisant ledit analogue sur un milieu de culture approprié et de la séparation du facteur VIII obtenu.

Les cellules ont, de préférence selon l'invention, été infectées par un vecteur viral recombinant tel que le virus de la vaccine qui assure l'expression du cADN de l'analogue du facteur VIII dans les cellules. Il s'agit, de préférence des cellules BHK21.

Mais, dans le cadre de la présente invention, on peut aussi transfecter les cellules par un vecteur d'intégration comportant le cADN de l'analogue du facteur VIII entouré des éléments nécessaires à son expression dans les cellules eucaryotes et un segment d'ADN favorisant l'intégration du vecteur dans l'ADN

EP 0 303 540 B1

cellulaire.

Dans ce cas, le cADN codant pour l'analogue du facteur VIII est, de préférence, inséré devant un gène codant pour un marqueur de sélection pour donner un produit de transcription bicistronique.

Les cellules transfectées sont, de préférence, des cellules CHO.

Enfin, la présente invention concerne une composition pharmaceutique comportant, à titre de principe actif, l'analogue du facteur VIII de la présente invention.

Une telle composition peut contenir, en outre, le facteur von Willebrand.

La composition pharmaceutique de la présente invention se présente de préférence, sous forme stérile injectable.

La présente invention sera mieux comprise à la lecture des exemples illustrés à l'aide des figures suivantes :

Figure 1 : Schéma de la maturation de la molécule du facteur VIII par clivages protéolytiques successifs (d'après Toole et al. 1984 (2) et Eaton et al. 1986 (7)).

Figure 2 : Schéma de la molécule du facteur VIII et des 2 délétions ΔI (aa 868 à 1562) et ΔII (aa 771 à 1666).

Les sites de clivage par la thrombine (IIa) et par une protéase inconnue (X₁ en position 1649) sont indiqués par des flèches.

La ligne inférieure représente la séquence du cADN avec les sites de restriction utilisés dans les diverses constructions.

Figure 3 : Schéma du vecteur d'expression du rFVIII utilisé pour transformer les cellules CHO.

Figure 4 : Cinétique d'activation par la thrombine des différentes molécules de facteur VIII :

pd = dérivé du plasma

r = molécule recombinante intacte

ΔII = molécule délétée ΔII

Note : La numérotation des nucléotides et des acides aminés est celle de Wood et al. (1).

Exemple 1 Construction de plasmides portant des dérivés du cADN du facteur VIII, délétés dans la région correspondant au domaine B.

Le plasmide de départ est le plasmide pTG1080 qui porte le cADN de facteur VIII, des nucléotides −64 à 8230, cloné dans le site SalI de pUC8 (Ce plasmide a été décrit dans le brevet français 86.08258).

A partir de ce plasmide, 2 dérivés ont été préparés, l'un (ΔI) comprenant une délétion dans le domaine B correspondant aux acides aminés 868 à 1562 ; l'autre (ΔII) correspondant à une délétion des acides aminés 771 à 1666. La construction ΔII abolit donc le site de clivage en position 1649.

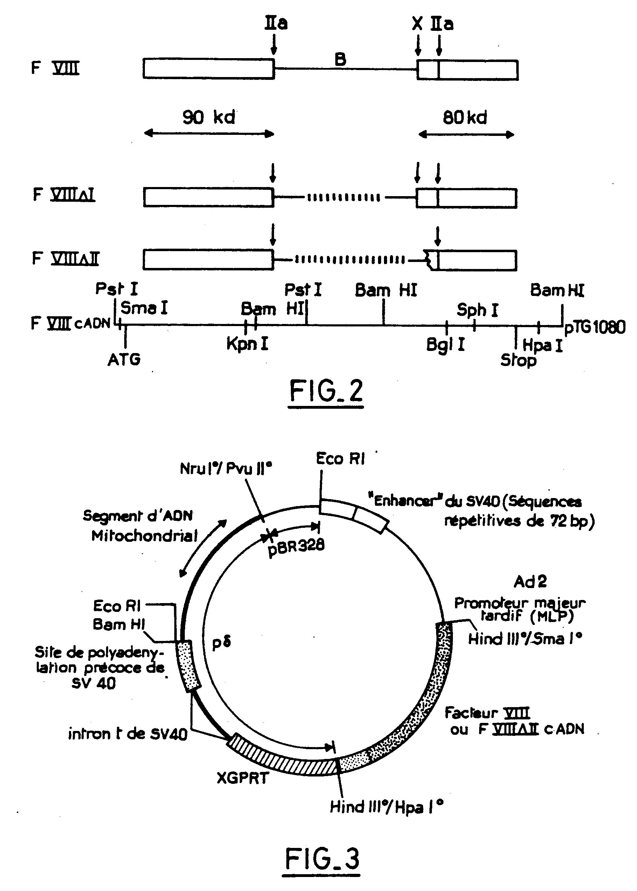

Les 2 constructions sont schématisées dans la figure 2.

a) Le plasmide pTG1080 a été digéré par l'enzyme PstI pour libérer un fragment de 2.7 kb comprenant l'extrémité 5′ correspondant aux nucléotides −64 à 2641 du cADN du facteur VIII.

Ce fragment PstI a été cloné dans le vecteur pTG1POLY (vecteur de clonage dérive de pML2 comportant une origine de réplication active dans E. coli et le gène de la β. lactamase, dans lequel on a inséré un "polylinker" présentant 12 sites de restriction uniques. Ce vecteur est identique au vecteur pTG14 décrit dans le brevet PCT−FR85/00096, à l'exception du "polylinker" qui remplace le "linker" HindIII. Dans cette construction, le site Bg1II du polylinker se trouve adjacent du site PstI (nucléotide 2641) de la séquence FVIII.

Ensuite on introduit dans ce site Bg1II, la position 3′ de la séquence codante du FVIII (nucléotides 4801 à 8230) sous forme de fragment BamHI également isolé à partir du pTG1080.

La construction présentant les 2 segments du FVIII dans l'orientation correcte est appelée pTG1501. La séquence du cADN FVIII portée par cette construction est appelée FVIIIΔI. La jonction PstI/Bg1II/BamHI ainsi formée préserve la phase de lecture du FVIII.

b) A partir du pTG1501, on récupère un fragment KpnI−SphI (correspondant aux nucléotides 1811 à 6580) pour l'introduire dans le vecteur M13TG131 (20) et le déléter des nucléotides 2367 à 5056, par la technique dite de "loop out mutagenesis" (17), avec un oligonucléotide de synthèse ayant la séquence suivante :

5′TCATTTCAACTGATATGGTGTCAGTCTT3′

Le vecteur délété est appelé M13TG1510 ; la séquence de cADN FVIII délétée est appelée FVIIIΔII.
L'analyse par séquençage confirme que la séquence est correcte.

3

Exemple 2 Expression des séquences FVIIIΔI et ΔII par des virus recombinants de la vaccine.

Dans le brevet français 86.08258 on a décrit le plasmide pTG1016 qui porte la séquence de cADN du facteur VIII en aval du promoteur du gène codant pour la protéine 7.5 K du virus de la vaccine et ce bloc d'expression est inséré dans le gène TK du virus de la vaccine.

Un dérivé du plasmide pTG1016, le pTG1030 (identique au précédent sauf une délétion Bg1II − PstI dans la région 5′ non traduite de la séquence FVIII) a été utilisé pour intégrer les constructions délétées, FVIIIΔI et ΔII, à la place de la séquence native, dans le vecteur conçu pour permettre l'intégration dans le génome du virus de la vaccine.

Le fragment BamHI − BglI (corespondant aux nucléotides 1864 à 6050) de la séquence native du pTG1030 est excisé et remplacé par le fragment BamHI − BglI du pTG1501 (FVIIIΔI) pour donner le pTG1506 ou par le fragment BamHI − BglI du M13TG1510 (FVIIIΔII) pour donner le pTG1507.

Les blocs d'ADN comprenant le gène TK de la vaccine interrompu par le cADN FVIIIΔ placé sous le promoteur 7.5 K de la vaccine, sont intégrés dans le génome du virus de la vaccine, par recombinaison homologue, selon la technique classique (18).

Les virus recombinants correspondants sont appelés VV.TG.FVIII1506 (FVIIIΔI) et VV.TG.FVIII1507 (FVIIIΔII).

Les virus recombinants sont utilisés pour infecter des tapis cellulaires de BHK21 ($2.10^6$ cellules) avec 1 ufp/cellule. Après 1 heure d'adsorption, les cultures sont lavées et renouvelées en milieu frais sans sérum, additionné de 1 % BSA et 1 mM $CaCl_2$. Des échantillons de milieu sont prélevés après 24 et 48 heures et dosés pour la quantité de facteur VIII présent, déterminée par dosage radioimmunométrique (FVIII:Ag) et pour l'activité procoagulante du facteur VIII (FVIII:C).

L'antigène FVIII ou FVIIIΔ est dosé "en sandwich" entre de l'immunoglobuline G du sérum de patient hémophile inhibiteur, adsorbée sur la paroi du tube à essai, et un anticorps monoclonal anti − facteur VIII, spécifique d'un épitope de la chaîne légère, radioactif, selon une méthode décrite par Lee et al. (21).

La mesure de l'activité du facteur VIII (FVIII:C) est réalisée classiquement par le temps partiel de thromboplastine activée (TPTA) (22).

Les résultats sont présentés dans le tableau suivant.

**Tableau 1 :** Détermination de la quantité de FVIII:Ag et de l'activité procoagulante FVIII:C dans le surnageant de cultures de BHK21 infectées avec les virus VV recombinants (résultats exprimés en mU/ml − seuil de détection = 1 mU/ml).

| VV recombinants | 24 heures | | 48 heures | |
|---|---|---|---|---|
| | FVIII:C | FVIII:Ag | FVIII:C | FVIII:Ag |
| VV.TG.1030 (FVIII) | 47.5 | 78.5 | 42.5 | 120.0 |
| VV.TG.1506-9 (FVIIIΔI) | 110.0 | 128.0 | 155.0 | 187.5 |
| VV.TG.1507-6 (FVIIIΔII) | 160.0 | 486.7 | 548.0 | 615.0 |

Les résultats montrent que les 2 molécules délétées sont biologiquement actives dans un test de coagulation sanguine et qu'on obtient une plus grande quantité de protéine avec les constructions FVIIIΔ qu'avec le FVIII intact (augmentation de 2 fois avec FVIIIΔI et de 5 fois avec FVIIIΔII).

De plus l'activité FVIII:C ne chute pas entre 24 et 48 heures alors qu'elle chute avec la molécule intacte. On sait que celle − ci doit être stabilisée par du facteur von Willebrand pour garder son activité (brevet français 86.08258).

Exemple 3 Etablissement de lignées cellulaires exprimant le facteur VIII ou le facteur VIIIΔII.

1) Construction de plasmides

Les séquences codantes du facteur VIII et du facteur VIIIΔII ont été introduites dans un vecteur, pTG384, conçu pour favoriser son intégration dans le génome de cellules de mammifères sous forme de multicopies. Ce vecteur a été décrit dans le brevet français 86.09043. Les éléments importants de ce vecteur (voir figure 3) sont :

a) un segment d'ADN mitochondrial murin qui favorise l'intégration des séquences exogènes d'ADN sous forme de plusieurs copies en tandem (15).

(b) une cassette d'expression comprenant les séquences "enhancer" (activateur de transcription) du SV40 (séquences répétitives de 72 pb), le promoteur majeur tardif (MLP) de l'Adénovirus 2, le gène codant pour le marqueur de sélection XGPRT (xanthine – guanine phosphoriboxyl transférase). Le vecteur est conçu pour permettre l'insertion en 5 ′ du gène XGPRT d'un cADN choisi, de manière à obtenir un produit de transcription bicistronique. Le vecteur comprend encore, en 3′ du XGPRT, l'intron du petit antigène t de SV40 et ses séquences de polyadénylation.

c) la propagation du vecteur dans E. coli est assurée par l'origine de réplication ColEl.

Les séquences codantes du FVIII ont été insérées dans le vecteur pTG384, dans le site unique HindIII (en amont du gène XGPRT). Les extrémités libérées par la digestion HindIII sont rendues franches par traitement au fragment Klenow de l'ADN polymérase I.

On récupère la séquence complète du FVIII à partir du plasmide pTG1080 (voir exemple 1) sous forme de fragment SmaI – HpaI (le site SmaI est situé dans le "polylinker" en amont de l'ATG initiateur et le site HpaI est situé au nucléotide 7434). La ligation de ce fragment dans le vecteur pTG384 ouvert par HindIII et traité à la Klenow donne le plasmide pTG1020.

Pour faire la construction analogue avec le gène délété du facteur VIII, on échange, dans le pTG1020, le fragment BamHI – BglI (nucléotides 1864 et 6050 du FVIII) avec le fragment FVIIIΔII, BamHI – BglI du M13TG1510, selon le même principe que pour la construction de pTG1507. Le vecteur pTG304 ayant inséré le FVIIIΔII est appelé pTG1509.

2) Transfection des cellules CHO avec les plasmides pTG1020 et pTG1509,

Des tapis de cellules CHO ont été transfectés avec l'ADN des plasmides pTG1020 (FVIII) ou pTG1509 (FVIIIΔII) par la méthode du précipité au phosphate de calcium (19), avec 5 ou 10 µg d'ADN par boîte de 8,5 cm de diamètre. 48 heures après la transfection, les cellules sont trypsinées, diluées et inoculées en milieu sélectif MEM α 2000 supplémenté avec de l'hypoxanthine (15 mg/l), de la thymidine (10 mg/l), de la xanthine (250 mg/l), de l'aminoptérine (0.2 mg/l), de l'acide mycophénolique (25 mg/l) et 10 % de sérum de veau foetal dialysé.

Après 2 semaines, les clones de cellules résistantes au milieu sélectif sont prélevés, mis en cultures en cupules de 1 ml puis de 2 ml. Quand les cellules atteignent 70 % de confluence, le milieu est enlevé, les tapis cellulaires sont lavés et renouvelés en milieu frais contenant 5 % de sérum inactivé (pour éviter un important bruit de fond dans les tests de coagulation ).

Après 24 heures le milieu est récolté et analysé pour la présence (FVIII:Ag) et l'activité (FVIII:C) du facteur VIII.

Plusieurs clones ont été obtenus. En première analyse, il apparaît que la plupart des clones exprimant le FVIII complet produisent moins de matériel que les clones exprimant le facteur VIIIΔII.

Deux clones ont été sélectionnés :

CHO – TG1020 – 22 – 12 qui exprime le FVIII complet

et CHO – TG1509 – 18 qui exprime le FVIIIΔII.

Les niveaux d'expression et d'activité FVIII sont présentés dans le tableau suivant :

Tableau 2

| Détermination de FVIII:Ag et FVIII:C (en mU) dans le surnageant des clones de CHO ($10^6$ cellules), récolté après 24 heures. | | |
|---|---|---|
| | FVIII:Ag | FVIII:C |
| CHO – TG1020 – 22 – 12<br>CHO – TG1509 – 18 | 63<br>504 | 60<br>500 |

On observe une production de facteur VIII$\Delta$II 10 fois supérieure à celle du facteur VIII complet, ce qui confirme les résultats observés dans le modèle vaccine.

Exemple 4 Activation par la thrombine des diverses molécules de facteur VIII natif et recombinants.

Différentes molécules de facteur VIII ont été comparées dans une cinétique d'activation par la thrombine : le facteur VIII natif dérivé du plasma et le facteur VIII recombinant, complet et $\Delta$II, exprimés dans les clones CHO.

L'activation est mesurée dans un test classique de coagulation (TPTA) après incubation en présence de quantité catalytique de thrombine.

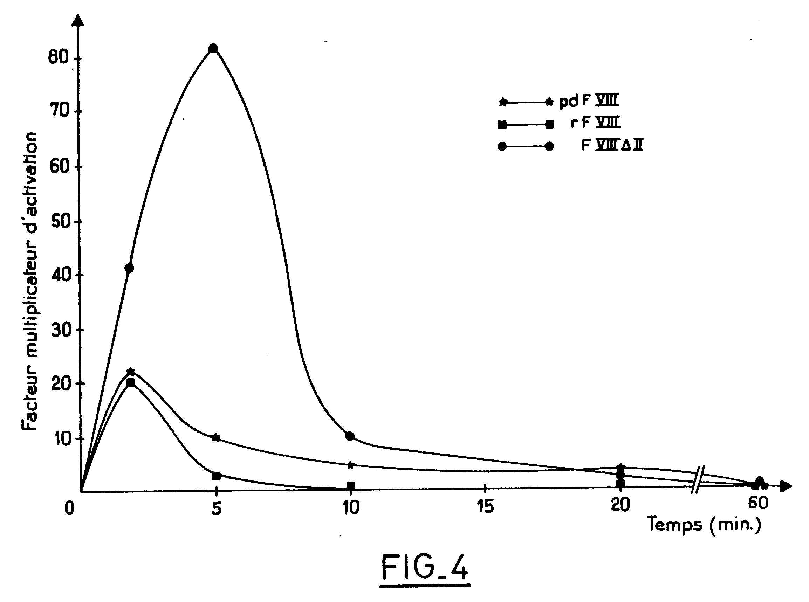

La figure 4 montre que le FVIII recombinant et le FVIII plasmatique sont activés de la même façon (d'un facteur 20) et suivant des cinétiques très voisines. Le FVIII$\Delta$II est activé beaucoup plus fort (d'un facteur 80 après 5 minutes d'incubation avec la thrombine).

Bien que le facteur VIII$\Delta$II soit beaucoup plus activé, il n'est pas activé plus rapidement que les 2 autres molécules (contrairement à ce qui à été observé par d'autres (9) avec leurs constructions facteur VIII$\Delta$) ce qui montre que le facteur VIII$\Delta$II n'est pas préactivé, ce qui est important en vue de son utilisation thérapeutique.

Le fait que le FVIII$\Delta$II n'est pas préactivé est confirmé par la bonne corrélation entre les niveaux de FVIII:Ag et de FVIII:C, à la fois dans le modèle vaccine et dans les lignées CHO.

Dépôt de souches représentatives de l'invention

Les souches suivantes ont été déposées à la Collection Nationale de Culture des Microorganismes, 25 Rue du Docteur Roux, Paris, le 24 juillet 1987 :
E. coli BJ5183/pTG1020, sous le n° I – 679
E. coli 5K/pTG1507, sous le n° I – 680
E. coli BJ5183/pTG1509, sous le n° I – 681.

REFERENCES

1) Wood, W.I. et al. Nature 312, 330 (1984).
2) Toole, J.J. et al. Nature 312, 342 (1984).
3) Truett, M.A. et al. DNA 4, 333 (1985).
4) Vehar, G.A. et al. Nature 312, 337 (1984).
5) Gitschier, J. et al. Nature 312, 326 (1984).
6) Fulcher, C.A. et al. Blood 61, 807 (1983).
7) Eaton, D.L. et al. Biochemistry 25, 505 (1986).
8) Toole, J.J. et al. Proc. Natl. Acad. Sci. USA 83, 5939 (1986).
9) Eaton, D.L. et al. Biochemistry 25, 8343 (1986).
10) Burke, R.L. et al. Biol. Chem. 261, 12574 (1986).
11) Pavirani, A. et al. In press.
12) Pavirani, A. et al. Bio/Technology (1987).
13) Mulligen, R.C. and Berg, P. Proc. Natl. Acad. Sci. USA 78, 2072 – 2076 (1981).
14) De La Salle, H. and Elkaim, R. Brevet Français N° 86.09043.
15) Lutfalla et al. Somatic cell and Mol. Genet. 11, 223 – 238.
16) Brinkhous, K.M. et al. Proc. Natl. Acad. Sci. USA 82, 8752 – 8756 (1985).
17) Zoller, M.J. and Smith, M. Methods Enzymol. 100B, 408 – 500 (1983).
18) Kieny, M.P. et al Nature 312, 163 – 166 (1984).

19) Graham, F.L. and van der Eb, A.J. Virology 52, 456 – 467 (1973).
20) Kieny, M.P., Lathe, R. and Lecocq, J.P. Gene 26, 91 – 99 (1983).
21) Lee, M.L., Maghalang, E.A. and Kingdon, M.J. Thromb, Res. 30, 511 – 519 (1983).
22) Girma, J.P., Lavergne, J.M., Meyer, D. and Larrien, M.J. Br. J. Haematol. 47, 269 – 282 (1981).

**Revendications**

1. Analogue du facteur VIII caractérisé en ce qu'il s'agit du composé délété des acides aminés 771 à 1666.

2. Procédé de préparation de l'analogue du facteur VIII selon la revendication 1, caractérisé en ce qu'on cultive des cellules eucaryotes produisant ledit analogue sur un milieu de culture approprié, et en ce qu'on sépare l'analogue du facteur VIII obtenu.

3. Procédé selon la revendication 2, caractérisé en ce que les cellules eucaryotes ont été infectées par un vecteur viral recombinant assurant l'expression du cADN codant pour l'analogue du facteur VIII.

4. Procédé selon la revendication 3, caractérisé en ce que le vecteur viral est le virus de la vaccine.

5. Procédé selon l'une quelconque des revendications 3 ou 4, caractérisé en ce que les cellules infectées sont des cellules BHK21.

6. Procédé selon la revendication 2, caractérisé en ce que les cellules eucaryotes ont été transfectées par un vecteur d'intégration comportant le cADN de l'analogue du facteur VIII et les éléments nécessaires à son expression dans lesdites cellules eucaryotes ainsi qu'un segment d'ADN favorisant l'intégration du vecteur dans l"ADN cellulaire.

7. Procédé selon la revendication 6, caractérisé en ce que le cADN codant pour l'analogue du facteur VIII est inséré devant un gène codant pour un marqueur de sélection pour donner un produit de transcription bicistronique.

8. Procédé selon les revendications 6 et 7, caractérisé en ce que les cellules eucaryotes sont des cellules CHO.

9. Composition pharmaceutique comportant à titre de principe actif l'analogue du facteur VIII selon la revendication 1.

10. Composition pharmaceutique selon la revendication 9, caractérisée en ce qu'elle contient en outre le facteur von Willebrand.

11. Composition pharmaceutique selon l'une quelconque des revendications 9 ou 10, caractérisée en ce qu'il s'agit d'une composition sous forme stérile injectable.

**Claims**

1. A factor VIII analog, which is the compound which has undergone deletion of amino acids 771 to 1666.

2. A process for preparing the factor VIII analog as claimed in claim 1, wherein eukaryotic cells that produce the said analog are cultured on a suitable culture medium, and where in the factor VIII analog obtained is separated.

3. The process as claimed in claim 2, wherein the eukaryotic cells have been infected with a recombinant viral vector that provides for the expression of the cDNA coding for the factor VIII analog.

4. The process as claimed in claim 3, wherein the viral vector is vaccinia virus.

5. The process as claimed in either of claims 3 and 4, wherein the infected cells are BHK21 cells.

**6.** The process as claimed in claim 2, wherein the eukaryotic cells have been transfected with an integration vector containing the cDNA of the factor VIII analog and the elements necessary for its expression in the said eukaryotic cells, as well as a DNA segment that promotes the integration of the vector in the cellular DNA.

**7.** The process as claimed in claim 6, wherein the cDNA coding for the factor VIII analog is inserted in front of a gene coding for a selection marker, to give a bicistronic transcription product.

**8.** The process as claimed in claims 6 and 7, wherein the eukaryotic cells are CHO cells.

**9.** A pharmaceutical composition containing, by way of active principle, the factor VIII analog as claimed in claim 1.

**10.** The pharmaceutical composition as claimed in claim 9, which contains, in addition, the von Willebrand factor.

**11.** The pharmaceutical composition as claimed in either of claims 9 and 10, which is a composition in sterile injectable form.

**Patentansprüche**

**1.** Analogon des Faktors VIII, dadurch gekennzeichnet, daß es sich dabei handelt um die deletierte (ausgelöschte) Verbindung der Aminosäuren 771 bis 1666.

**2.** Verfahren zur Herstellung des Analogons des Faktors VIII nach Anspruch 1, dadurch gekennzeichnet, daß man Eukaryonten – Zellen kultiviert, die das Analogon auf einem geeigneten Kultur – Medium bilden, und daß man das erhaltene Analogen des Faktors VIII abtrennt.

**3.** Verfahren nach Anspruch 2, dadurch gekennzeichnet, daß die Eukaryonten – Zellen durch einen rekombinanten Virus – Vektor infiziert worden sind, um die Expression der cDNA zu gewährleisten, die für das Analogon des Faktors VIII codiert.

**4.** Verfahren nach Anspruch 3, dadurch gekennzeichnet, daß es sich bei dem Virus – Vektor um das Vaccine – Virus handelt.

**5.** Verfahren nach einem der Ansprüche 3 oder 4, dadurch gekennzeichnet, daß die infizierten Zellen BHK21 – Zellen sind.

**6.** Verfahren nach Anspruch 2, dadurch gekennzeichnet, daß die Eukaryonten – Zellen durch einen Integrations – Vektor transfektiert worden sind, der umfaßt die cDNA des Analogons des Faktors VIII und die für seine Expression in die genannten Eukaryonten – Zellen erforderlichen Elemente sowie ein DNA – Segment, das die Integration des Vektors in die Zell – DNA begünstigt.

**7.** Verfahren nach Anspruch 6, dadurch gekennzeichnet, daß die für das Analogon des Faktors VIII codierende cDNA inseriert wird vor einem Gen, das für einen Selektionsmarker codiert, um ein bicistronisches Transkriptionsprodukt zu ergeben.

**8.** Verfahren nach den Ansprüchen 6 und 7, dadurch gekennzeichnet, daß die Eukaryonten – Zellen CHO – Zellen sind.

**9.** Pharmazeutische Zusammensetzung, die als aktives Prinzip (Wirkstoff) das Analogen des Faktors VIII nach Anspruch 1 enthält.

**10.** Pharmazeutische Zusammensetzung nach Anspruch 9, dadurch gekennzeichnet, daß sie außerdem den von Willebrand – Faktor enthält.

**11.** Pharmazeutische Zusammensetzung nach einem der Ansprüche 9 oder 10, dadurch gekennzeichnet, daß es sich dabei um eine Zusammensetzung in einer injizierbaren sterilen Form handelt.

FIG.1

FIG.2

FIG.3

FIG_4

EP 0 303 540 B1